(19) **Europäisches Patentamt / European Patent Office / Office européen des brevets**

(11) **EP 4 410 282 B1**

(12) **EUROPEAN PATENT SPECIFICATION**

(45) Date of publication and mention
of the grant of the patent:
**24.06.2026 Bulletin 2026/26**

(21) Application number: **22889923.3**

(22) Date of filing: **31.10.2022**

(51) International Patent Classification (IPC):
**A61K 9/70** *(2006.01)*        **A61K 47/32** *(2006.01)*

(52) Cooperative Patent Classification (CPC):
**A61K 9/7084**

(86) International application number:
**PCT/JP2022/040616**

(87) International publication number:
**WO 2023/080103 (11.05.2023 Gazette 2023/19)**

(54) **ADHESIVE PATCH WITH COVER MATERIAL**

KLEBEPFLASTER MIT ABDECKMATERIAL

TIMBRE ADHÉSIF AVEC MATÉRIAU DE RECOUVREMENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.11.2021 JP 2021180374**

(43) Date of publication of application:
**07.08.2024 Bulletin 2024/32**

(73) Proprietor: **Hisamitsu Pharmaceutical Co., Inc.
Tosu-shi, Saga 841-0017 (JP)**

(72) Inventors:
• **KURIBAYASHI Mitsuru
Tosu-shi, Saga 841-0017 (JP)**
• **TAKEO Masafumi
Tosu-shi, Saga 841-0017 (JP)**

(74) Representative: **Prüfer & Partner mbB
Patentanwälte · Rechtsanwälte
Sohnckestraße 12
81479 München (DE)**

(56) References cited:
EP-A1- 3 263 097          WO-A1-2015/087927
WO-A1-2020/071205        WO-A1-2020/175395
WO-A1-2021/201152        WO-A1-2021/201152

**Description**

Technical Field

The present invention relates to an adhesive patch with a cover material.

Priority is claimed on Japanese Patent Application No. 2021-180374, filed on November 4, 2021.

Background Art

In the related art, transdermal and transmucosal absorption preparations have been developed as preparations for administering drugs into living bodies. Particularly, an adhesive patch which is easy to handle and can maintain an effective blood concentration of a drug for a long period of time has attracted attention.

In the case where a part or all of the adhesive patch is peeled off from the skin during the administration thereof, the blood concentration of the drug cannot be maintained.

In order to prevent this, a cover material is known that prevents an adhesive patch from peeling off from the skin and maintains a state in which the adhesive patch is in contact with the skin by attaching the cover material to the skin to cover the adhesive patch. For example, Patent Document 1 discloses a cover sheet that has both specific adhesive strength and stretchable properties to supplement skin adhesive strength of an adhesive patch preparation and to ensure adhesiveness of the adhesive patch preparation to the skin surface, and a method of using it.

Document of Related Art

Patent Document

Patent Document 1: Japanese Unexamined Patent Application, First Publication No. H11-343232
Patent Document 2: EP3263097

Summary of Invention

Problems to be Solved by Invention

The problem with cover materials of the related art including Patent Document 1 is that adhesive properties may be insufficient, or an adhesive may gradually exude from the preparation due to external forces not intended by the user such as movements or vibrations of the user during application, resulting in unpleasant stickiness. Patent Document 2 discloses a method for producing a gel patch comprising an adhesive mass layer on a backing fabric.

The present inventors have conducted various studies focusing on a backing for a cover material and an adhesive. As a result, the present inventors have found that it is possible to provide an adhesive patch with a cover material capable of solving all of the above-described problems by combining a specific backing and adhesive, thereby completing the present invention.

In view of the above-described circumstances, an object of the present invention is to provide an adhesive patch with a cover material that suitably maintains a state in which the adhesive patch is in contact with the skin, resulting in excellent adhesive properties of the cover material and suppression of unpleasant stickiness.

Means for Solving Problems

The present invention provides an adhesive patch with a cover material including an adhesive patch part that includes a backing and a drug layer formed on the backing; and a cover part that includes a cover layer and a cover adhesive layer formed on the cover layer, in which the cover adhesive layer is attached to the backing to cover the adhesive patch part.

The cover layer is formed of a non-woven fabric, and has an elongation rate of 50% to 300% in a flow direction and a width direction.

The cover adhesive layer is made of a rubber-based adhesive in which a main adhesive base is a rubber-based adhesive base, and has a loss tangent ($\tan\delta$) of 0.7 to 0.95 at 32°C and 0.1 Hz.

Effects of Invention

According to the present invention, it is possible to provide an adhesive patch with a cover material that suitably maintains a state in which the adhesive patch is in contact with the skin, resulting in excellent adhesive properties and at the same time suppressing unpleasant stickiness.

Brief Description of Drawings

FIG. 1 is a plan view showing an adhesive patch with a cover material according to an embodiment of the present invention.
FIG. 2 is a cross-sectional view taken along a line I-I of FIG. 1.

Embodiments of Invention

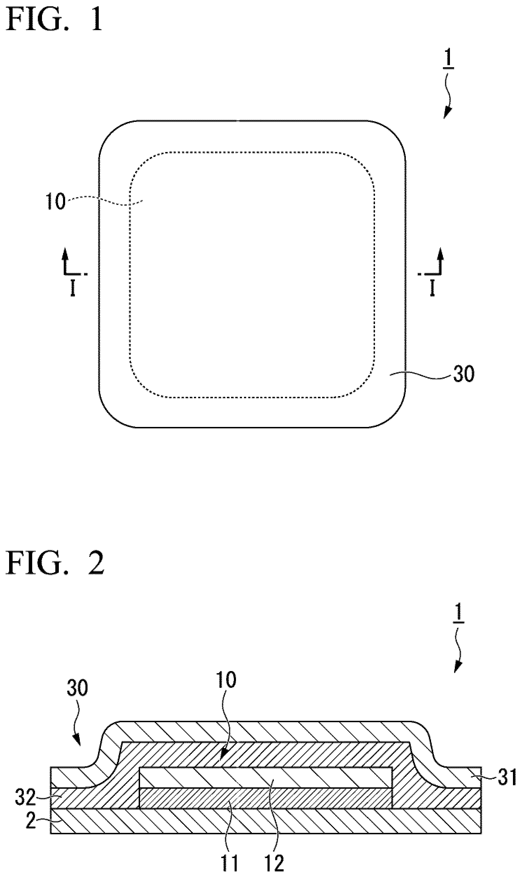

An embodiment of the present invention will be described. FIG. 1 is a plan view of an adhesive patch 1 with a cover material according to the present embodiment, and FIG. 2 is a cross-sectional view taken along a line I-I of FIG. 1.

The adhesive patch 1 with a cover material includes an adhesive patch part 10 containing a drug and a cover part 30 attached to the adhesive patch part 10.

As shown in FIG. 2, the adhesive patch part 10 includes a drug layer 11 and a backing 12. The drug layer 11 contains a desired drug, and upon contact with the skin, the drug is transdermally absorbed into the user's body.

Since the adhesive patch 1 with a cover material maintains a contact state between the drug layer 11 and the skin mainly through the adhesion of the cover part 30, the drug layer 11 may or may not have adhesiveness; but it is preferable that the drug layer 11 have adhesiveness to ensure that the contact state between the drug layer 11 and the skin can be suitably maintained and that continuous administration of the drug can be further ensured.

The drug layer 11 contains a base and a drug. Among the bases, examples of the adhesive base for imparting adhesiveness to the drug layer 11 include a rubber-based adhesive base, an acrylic adhesive base, and a silicone-based adhesive base. The adhesive base is preferably one or more selected from the group consisting of a rubber-based **adhesive** base, an acrylic adhesive base, and a silicone-based adhesive base. The adhesive base may be any of a rubber-based adhesive base, an acrylic adhesive base, and a silicone-based adhesive base, or may be a combination thereof. The total content of the adhesive base can be 10% by mass to 95% by mass and may be 20% by mass to 90% by mass, based on the total mass of the drug layer 11.

The drug layer 11 is preferably substantially free of water (non-aqueous). In the present invention, the phrase "substantially free of water" means that there is no step of intentionally incorporating water into the drug layer during the production process, but does not exclude moisture in the air or the like incorporated during the production process or moisture absorbed from sweat or the like during application of the adhesive patch to the skin.

Examples of the rubber-based adhesive base include, but are not particularly limited to, a styrene-isoprene block copolymer, a styrene-isoprene-styrene block copolymer, a polyisoprene, a polyisobutylene, a polybutene, a styrene-butadiene block copolymer, a styrene-butadiene-styrene block copolymer, a natural rubber, an alkyl vinyl ether (co) polymer, and a polybutadiene. From the viewpoint of adhesiveness, spreadability, and cohesion, a styrene-butadiene-styrene block copolymer and a polyisobutylene are particularly preferable.

Among rubber-based adhesive bases, specific examples of the styrene-isoprene-styrene block copolymer include Quintac (registered trademark) 3570C (trade name, manufactured by Zeon Corporation), SIS5002, SIS5229, and SIS5505 (trade names, manufactured by JSR Corporation), and SIBSTAR (registered trademark) T102 (trade name, manufactured by Kaneka Corporation). Examples of the polyisobutylene also include so-called butyl rubber (isobutylene-isoprene rubber). Specific examples of the polyisobutylene include Oppanol (registered trademark) N50, N80, N100, N150, B11, B12, B50, B80, B100, B120, B150, and B220 (trade names, manufactured by BASF SE), JSR (registered trademark) Butyl 065, 268, and 365 (trade names, manufactured by JSR Corporation), X_Butyl (registered trademark) RB100, 101-3, 301, and 402 (trade names, manufactured by Arlanxeo Company), Exxon (registered trademark) Butyl 065, 065S, 068, 068S, 268, 268S, 365, and 365S (trade names, manufactured by Exxon Mobil Corporation), and Butyl 065, 268, and 365 (trade names, manufactured by Nippon Butyl Co., Ltd.).

In the case where the drug layer 11 contains a rubber-based adhesive base, the content of the rubber-based adhesive base can be 10% by mass to 95% by mass and may be 20% by mass to 90% by mass, based on the total mass of the drug layer 11.

The acrylic adhesive base is not particularly limited and is, for example, a (co)polymer of one or two or more types of (meth)acrylic acid alkyl esters. Examples of the (meth)acrylic acid alkyl ester include butyl (meth)acrylate, isobutyl (meth)acrylate, hexyl (meth)acrylate, octyl (meth)acrylate, 2-ethylhexyl (meth)acrylate, and decyl (meth)acrylate. In the present specification, the term "(meth)acrylic acid" means either or both of acrylic acid and methacrylic acid, and similar expressions are similarly defined.

The acrylic adhesive base is not particularly limited, and may be a copolymer formed from a (meth)acrylic acid alkyl ester (main monomer) and a comonomer. Examples of the main monomer include methyl (meth)acrylate, ethyl (meth)acrylate, butyl (meth)acrylate, hexyl (meth)acrylate, heptyl (meth)acrylate, octyl (meth)acrylate, and 2-ethylhexyl (meth)acrylate. Among these monomers, one type of monomer may be used alone, or two or more types may be used in combination. A comonomer may be any component as long as it can be copolymerized with the (meth)acrylic acid alkyl ester. Examples of the comonomer include a (meth)acrylic acid hydroxyalkyl ester, ethylene, propylene, styrene, vinyl acetate, N-vinylpyrrolidone, (meth)acrylic acid, and a (meth)acrylic acid amide. As for the comonomer, only one type of the above-described comonomers may be used, or two or more types of the above-described comonomers may be used.

Specific examples of such an acrylic adhesive base include DURO-TAK series (manufactured by Henkel AG & Co. KGaA) such as DURO-TAK (registered trademark) 387-2510, DURO-TAK (registered trademark) 87-2510, DURO-TAK (registered trademark) 387-2287, DURO-TAK (registered trademark) 87-2287, DURO-TAK (registered trademark) 87-4287, DURO-TAK (registered trademark) 387-2516, DURO-TAK (registered trademark) 87-2516, DURO-TAK (registered trademark) 87-2074, DURO-TAK (registered trademark) 87-900A, DURO-TAK (registered trademark) 87-901A, DURO-TAK (registered trademark) 87-9301, and DURO-TAK (registered trademark) 87-4098; GELVA series (manufactured by Henkel AG & Co. KGaA) such as GELVA (registered trademark) GMS 788, GELVA (registered trademark) GMS 3083, and GELVA (registered trademark) GMS 3253; MAS series (manufactured by CosMED Pharmaceutical Co., Ltd.) such as MAS811 (trade name) and MAS683 (trade name); Eudragit (registered trademark) series (manufactured by Evonik Industries AG), Nikasol (registered trademark) series (manufactured by Nippon Carbide Industries Co., Inc.), and Ultrasol (registered trademark) series (manufactured by Aica Kogyo Co., Ltd.).

In the case where the drug layer 11 contains an acrylic adhesive base, the content of the acrylic adhesive base can be 10% by mass to 95% by mass and may be 20% by mass to 90% by mass based on the total mass of the drug layer 11.

The silicone-based adhesive base is not particularly limited and may be a compound having an organopolysiloxane skeleton. Examples of the silicone-based adhesive base include dimethylpolysiloxane, polymethylvinylsiloxane, and polymethylphenylsiloxane.

Specific examples of the silicone-based adhesive base include MD series (manufactured by Dupont Toray Specialty Materials K.K.) such as MD7-4502 Silicone Adhesive and MD7-4602 Silicone Adhesive; BIO-PSA series (manufactured by Dupont Toray Specialty Materials K.K.) such as BIO-PSA (registered trademark) 7-4301 Silicone Adhesive, BIO-PSA (registered trademark) 7-4302 Silicone Adhesive, BIO-PSA (registered trademark) 7-4201 Silicone Adhesive, BIO-PSA (registered trademark) 7-4202 Silicone Adhesive, BIO-PSA (registered trademark) 7-4101 Silicone Adhesive, BIO-PSA (registered trademark) 7-4102 Silicone Adhesive, BIO-PSA (registered trademark) 7-4601 Silicone Adhesive, BIO-PSA (registered trademark) 7-4602 Silicone Adhesive, BIO-PSA (registered trademark) 7-4501 Silicone Adhesive, BIO-PSA (registered trademark) 7-4502 Silicone Adhesive, BIO-PSA (registered trademark) 7-4401 Silicone Adhesive, and BIO-PSA (registered trademark) 7-4402 Silicone Adhesive; Dow Corning (registered trademark) 7-9800A, Dow Corning (registered trademark) 7-9800B, Dow Corning (registered trademark) 7-9700A, and Dow Corning (registered trademark) 7-9700B.

In the case where the drug layer 11 contains a silicone-based adhesive base, the content of the silicone-based adhesive base can be 10% by mass to 95% by mass and may be 20% by mass to 90% by mass based on the total mass of the drug layer 11.

The drug is not particularly limited as long as it has a molecular weight that allows transdermal absorption and can be administered for a long period of time, for example, about one week.

The drugs to which the present invention can be applied are not particularly limited in terms of their drug efficacy, and include, for example, a hypnotic or sedative agent (flurazepam, rilmazafone, phenobarbital, amobarbital, medetomidine, dexmedetomidine, or the like), an antipyretic anti-inflammatory analgesic (butorphanol, perisoxal, acetaminophen, mefenamic acid, diclofenac sodium, aspirin, alclofenac, ketoprofen, flurbiprofen, naproxen, pyroxycam, pentazocine, indomethacin, felbinac, glycol salicylate, aminopyrin, loxoprofen, meloxicam, lornoxicam, or the like), a steroidal anti-inflammatory agent (hydrocortisone, prednisolone, dexamethasone, betamethasone, or the like), a stimulant or anti-hypnotic agent (methamphetamine, amphetamine, methylphenidate, or the like), a psychoneurotic agent (imipramine, diazepam, sertraline, fluvoxamine, paroxetine, citalopram, fluoxetine, alprazolam, haloperidol, clomipramine, amitriptyline, desipramine, amoxapine, maprotiline, mianserin, setiptiline, trazodone, lofepramine, milnacipran, duloxetine, venlafaxine, chlorpromazine, thioridazine, diazepam, meprobamate, etizolam, risperidone, asenapine, or the like), a hormonal agent (estradiol, estriol, progesterone, norethisterone, methenolone, testosterone, or the like), a local anesthetic (lidocaine, procaine, tetracaine, dibucaine, propitocaine, or the like), an agent for urinary organs (oxybutynin, tamsulosin, propiverine, imidafenacin, solifenacin, tolterodine, or the like), a skeletal muscle relaxant (tizanidine, eperisone, pridinol, suxamethonium, or the like), an agent for reproductive organs (ritodrine, meluadrine, or the like), an antiepileptic agent (sodium valproate, clonazepam, carbamazepine, or the like), an agent for autonomous nerves (carpronium, neostigmine, bethanechol, or the like), an anti-Parkinson's agent (pergolide, bromocriptine, trihexyphenidyl, amantadine, talipexole, cabergoline, droxidopa, biperiden, selegiline, ropinirole, or the like), a diuretic agent (hydroflumethiazide, furosemide, or the like), a respiratory stimulant (lobeline, dimorpholamine, naloxone, or the like), an antimigraine agent (dihydroergotamine, sumatriptan, ergotamine, flunarizine, cyproheptadine, or the like), an antihistamine agent (clemastine, diphenhydramine, chlorpheniramine, diphenylpyraline, promethazine, or the like), a bronchodilator (tulobuterol, procaterol, salbutamol, clenbuterol, fenoterol, terbutaline, isoprenaline, formoterol, or the like), a cardiotonic agent (isoprenaline, dopamine, or the like), a coronary vasodilator (diltiazem, verapamil, isosorbide, nitroglycerin, nicorandil, or the like), a peripheral vasodilator (nicametate, tolazoline, or the like), a smoking cessation aid (nicotine, varenicline, or the like), a cardiovascular agent (flunarizine, nicardipine, nitrendipine, nisoldipine, felodipine, amlodipine, nifedipine, nilvadipine, manidipine, benidipine, enalapril, temocapril, alacepril, imidapril, cilazapril, lisinopril, captopril, trandolapril, perindopril erbumine, atenolol, bisoprolol, metoprolol, betaxolol, arotinolol, celiprolol, carvedilol, carteolol, bevantolol, valsartan, candesartan cilexetil, losartan potassium, clonidine, or the like), an antiarrhythmic agent (propranolol, alprenolol, procainamide, mexiletine, nadolol, disopyramide, or the like), an anti-malignant ulcer agent (cyclophosphamide, fluorouracil, tegafur, procarbazine, ranimustine, irinotecan, fluridine, or the like), an antilipemic agent (pravastatin, simvastatin, bezafibrate, probucol, or the like), a hypoglycemic agent (glibenclamide, chlorpropamide, tolbutamide, glymidine sodium, glybuzole, buformin, or the like), a therapeutic agent for peptic ulcer (proglumide, cetraxate, spizofurone, cimetidine, glycopyrronium, or the like), a choleretic agent (ursodesoxycholic acid, osalmid, or the like), a gastrointestinal prokinetic agent (domperidone, cisapride, or the like), an agent for liver diseases (tiopronin or the like), an anti-allergic agent (ketotifen, azelastine, emedastine, or the like), an antiviral agent (acyclovir or the like), an antivertigo agent (betahistine, diphenidol, or the like), an antibiotic (cephaloridine, cefdinir, cefpodoxime proxetil, cefaclor, clarithromycin, erythromycin, methylerythromycin, kanamycin, cycloserine, tetracycline, benzylpenicillin potassium, propicillin potassium, cloxacillin sodium, ampicillin sodium, bacampicillin, carbenicillin sodium, chloramphenicol, or the like), an agent for habitual addiction (cyanamide or the like), an anorectic agent (mazindol or the like), a chemotherapeutic agent (isoniazid, ethionamide, pyrazinamide, or the like), a blood coagulation accelerator (ticlopidine, warfarin potassium, or the like), an anti-Alzheimer's agent (physostigmine, donepezil, tacrine, arecoline, xanomeline, galantamine, rivastigmine, or the like), a serotonin receptor antagonist antiemetic agent (ondansetron, granisetron, ramosetron, azasetron, or the like), an antipodagric agent (colchicine, probenecid, sulfinpyrazone, or the like), a narcotic analgesic (morphine, codeine, cocaine, pethidine, fentanyl, or the like), and an antifungal agent (terbinafine, butenafine, amorolfine, neticonazole, miconazole, luliconazole, itraconazole, liranaftate, or the like). One of the above-described drugs may be used alone, or two or more may be used in combination.

The content of the drug can be 0.1% by mass to 30% by mass and may be 1% by mass to 20% by mass, based on the total mass of the drug layer 11.

The drug layer 11 may contain an additive in addition to the base and the drug. Examples of the additive include a tackifier resin, a plasticizer, an absorption enhancer, a solubilizing agent, a stabilizing agent, a filler, an adsorbent, a desalting agent, a pH-adjusting agent, and a fragrance. Only one additive may be used, or two or more may be used.

The tackifier resin is a component that adjusts the adhesiveness of the drug layer. Examples of the tackifier resin include a petroleum-based resin, a terpene-based resin, a rosin-based resin, a phenol-based resin, and a xylene-based resin. Examples of the petroleum-based resin include an alicyclic petroleum resin (an alicyclic saturated hydrocarbon resin or the like), an aliphatic petroleum resin (an aliphatic hydrocarbon resin or the like), and an aromatic petroleum resin. More specific examples of the petroleum-based resin include Arkon P-70, Arkon P-85, Arkon P-90, Arkon P-100, Arkon P-115, Arkon P-125, Arkon M-70, Arkon M-85, Arkon M-90, Arkon M-100, Arkon M-115, and Arkon M-125 (all trade names, manufactured by Arakawa Chemical Industries, Ltd.), and Escorez 8000 (trade name, manufactured by Esso Petrochemical Co., Ltd.). Examples of the terpene-based resin include a pinene polymer (an $\alpha$-pinene polymer, a $\beta$-pinene polymer, or the like), a terpene polymer, a dipentene polymer, a terpene-phenol polymer, an aromatic modified terpene polymer, and a pinene-phenol copolymer. More specific examples of the terpene-based resin include YS resin (YS resin PXN (1150N or 300N), YS resin PX1000, YS resin TO125, YS resin TO105, or the like), Clearon P105, Clearon M115, and Clearon K100 (all trade names, manufactured by Yasuhara Chemical Co., Ltd.), and Tamanol 901 (trade name, manufactured by Arakawa Chemical Industries, Ltd.). Examples of the rosin-based resin include a hydrogenated rosin glycerin ester, an ultra-light color rosin, an ultra-light color rosin ester, and an acid-modified ultra-light color rosin. More specific examples of the rosin-based resin include Pinecrystal (KE-311, PE-590, KE-359, KE-100, or the like) (trade names, manufactured by Arakawa Chemical Industries Ltd.). One of the above-described tackifier resins may be used alone, or two or more may be used in combination. In the case where the drug layer contains a tackifier resin, the content of the tackifier resin can be 15% by mass to 80% by mass and may be 25% by mass to 65% by mass, based on the total mass of the drug layer.

Examples of the plasticizer include paraffin oils (liquid paraffin and the like), squalane, squalene, vegetable oils (olive oil, camellia oil, castor oil, tall oil, peanut oil, spearmint oil, eucalyptus oil, jojoba oil, camphor white oil, sunflower seed oil, orange oil, and the like), fats and oils (dibutyl phthalate, dioctyl phthalate, and the like), and liquid rubbers (liquid polybutene, liquid isoprene rubber, and the like). A preferred plasticizer is liquid paraffin. One plasticizer may be used alone, or two or more types may be used in combination. In the case where the drug layer contains a plasticizer, the content of the plasticizer is, for example, 3% by mass to 50% by mass, 5% by mass to 30% by mass, or 7% by mass to 20% by mass, based on the total mass of the drug layer.

The transdermal absorption enhancer may be any compound that is recognized in the related art to have a transdermal absorption promoting effect on the skin. Examples of the transdermal absorption enhancer include organic acids, fatty acids having 6 to 20 carbon chains, fatty alcohols, fatty acid esters, amides, ethers, aromatic organic acids, aromatic alcohols, aromatic organic acid esters and ethers (these compounds may be saturated or unsaturated compounds and may be cyclic, linear, or branched compounds), lactic acid esters, acetic acid esters, monoterpene-based compounds, sesquiterpene-based compounds, Azone, Azone derivatives, pyrothiodecane, glycerin fatty acid esters, propylene glycol fatty acid esters, sorbitan fatty acid esters (Span family), polysorbate-based compounds (Tween family), polyethylene glycol fatty acid esters, polyoxyethylene hydrogenated castor oil-based compounds (HCO family), polyoxyethylene alkyl ethers, sucrose fatty acid esters, and vegetable oils.

Examples of other transdermal absorption enhancers include lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methyl laurate, hexyl laurate, diethanolamide laurate, isopropyl myristate, myristyl myristate, octyldodecyl myristate, cetyl palmitate, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, l-menthol, borneol, d-limonene, isoeugenol, isoborneol, nerol, dl-camphor, glycerin monocaprylate, glycerin monocaprate, glycerin monolaurate, glycerin monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, polyoxyethylene hydrogenated castor oil, pyrothiodecane, and olive oil. Two or more types of such transdermal absorption enhancers may be mixed and used. The content of the transdermal absorption enhancer is, for example, preferably 0.1% to 20% by mass and more preferably 0.5% to 15% by mass, based on the total mass of the drug layer.

Although the solubilizing agent depends on the types of solutes to be dissolved, examples of the solubilizing agent include fatty acids (for example, capric acid, oleic acid, and linoleic acid), fatty acid esters (for example, isopropyl myristate and isopropyl palmitate), fatty acid derivatives (for example, propylene glycol monolaurate and diethanolamide laurate), fatty acid glycerin esters (for example, glycerin monolaurate and glycerin monooleate), polyhydric alcohol esters of fatty acids (for example, sorbitan monolaurate), aliphatic alcohols (for example, octyldodecanol, isostearyl alcohol, and oleyl alcohol), polyhydric alcohols (for example, propylene glycol, dipropylene glycol, and polyethylene glycol), pyrrolidone derivatives (for example, N-methyl-2-pyrrolidone), organic acids (for example, acetic acid and lactic acid), and organic acid salts (for example, sodium acetate and sodium lactate). As for the solubilizing agent, one of the above-described solubilizing agents may be used alone, or two or more may be used in combination. The content of the solubilizing agent is, for example, preferably 0% to 20% by mass and more preferably 0.5% to 15% by mass, based on the total mass of the drug layer.

Examples of the stabilizing agent include tocopherol and ester derivatives of tocopherol, ascorbic acid, ascorbic acid stearyl ester, nordihydroguaiaretic acid, dibutylhydroxytoluene, and butylhydroxyanisole. One of the above-described stabilizing agents may be used alone, or two or more types may be used in combination. The content of the stabilizing agent is preferably, for example, 0.1% to 5% by mass based on the total mass of the drug layer.

Examples of the filler include inorganic compounds such as silica, aluminum oxide, aluminum hydroxide, zinc oxide, titanium oxide, talc, clay, kaolin, glass, barium sulfate, calcium carbonate, hydroxyapatite, and ceramics; and organic compounds such as cellulose, silk, polyester, polyolefin, polyacrylic acid ester, polymethacrylic acid ester, and polystyrene. As for the filler, one of the above-described fillers may be used alone, or two or more may be used in combination. The content of the filler is preferably, for example, 0.1% to 5% by mass based on the total mass of the drug layer.

Examples of the adsorbent include inorganic and/or organic substances having hygroscopic properties. More specific examples of the adsorbent include minerals such as talc, kaolin, and bentonite; silicon compounds such as fuined silica (Aerosil (registered trademark) and the like) and hydrous silica; metal compounds such as zinc oxide and dry aluminum hydroxide gel; weak acids such as lactic acid and acetic acid; sugars such as dextrin; and high molecular weight polymers such as polyvinylpyrrolidone, aminoalkyl methacrylate copolymers, crospovidone, carboxyvinyl polymers, and butyl methacrylate methyl methacrylate copolymers. As for the adsorbent, one type of the above-described adsorbents may be used alone, or two or more types of the above-described adsorbents may be used in combination. The content of the adsorbent is, for example, preferably 0.1 % to 20% by mass and more preferably 0.5% to 10% by mass, based on the total mass of the drug layer.

Examples of the desalting agent and the pH-adjusting agent include, but are not particularly limited to, sodium acetate (including anhydrous sodium acetate), sodium hydroxide, potassium hydroxide, magnesium hydroxide, sodium hydrogen carbonate, potassium hydrogen carbonate, sodium citrate, and sodium lactate. As for the desalting agent and the pH-adjusting agent, one type of the above-described desalting agents and pH-adjusting agents may be used alone, or two or more types of the above-described desalting agents and pH-adjusting agents may be used in combination. The content of the desalting agent and the pH-adjusting agent is preferably, for example, 0.1 % to 5% by mass based on the total mass of the drug layer.

The mass of the paste of the drug layer is not particularly limited, and is preferably 20 to 500 g/m$^2$ and more preferably 50 to 300 g/m$^2$.

The backing 12 is not particularly limited as long as it can support the drug layer 11, but it is preferably one through which the drug contained in the drug layer 11 does not permeate or hardly permeates. As the material for the backing 12, films and the like consisting of various synthetic resins are exemplary examples. Examples of the material for such a backing include synthetic resins, for example, polyesters such as polyethylene terephthalate (PET), polybutylene terephthalate, and polyethylene naphthalate; polyolefins such as polyethylene and polypropylene; polyurethanes; and ethylene-vinyl acetate copolymers, metals such as aluminum, and paper. In addition, examples of the form of the backing consisting of such a material include a film; a sheet such as a foamed sheet, a porous sheet, or a microporous sheet; a fabric such as a woven fabric, a knitted fabric, or a non-woven fabric; a foil; and a laminate thereof. From the viewpoint of further suppressing the migration of the drug from the drug layer to the cover adhesive layer, the backing is preferably one that does not allow the drug to permeate therethrough. Particularly, from the viewpoint of providing excellent flexibility and drug impermeability, the backing is preferably a film made of polyester.

The thickness of the backing 12 is not particularly limited, and is usually preferably about 2 to 600 $\mu$m. The thickness of the backing 12 is more preferably 5 to 100 $\mu$m, from the viewpoint that it can be brought into sufficiently close contact with the cover part 30 and can be applied to the skin for a longer period of time while maintaining sufficient strength.

The cover part 30 includes a cover layer 31 and a cover adhesive layer 32 and is attached to the adhesive patch part 10 from the backing 12 side. In the plan view of the adhesive patch 1 with a cover material shown in FIG. 1, the cover part 30 completely covers the adhesive patch part 10, and a part of the cover adhesive layer 32 is attached to the backing 12.

The cover layer 31 according to the present embodiment is formed of a non-woven fabric. The material for the fiber constituting the non-woven fabric is not particularly limited, and examples thereof include polyolefins such as polyethylene, polypropylene, and polybutadiene; polyesters such as an ethylene vinyl acetate copolymer, polyvinyl chloride, PET, polyethylene naphthalate, and polybutylene terephthalate; polyamides such as nylon; polyurethanes; and natural fibers such as silk, animal hair (for example, wool), and celluloses such as cotton. Particularly, a synthetic resin such as PET is suitable. The non-woven fabric may be configured by mixing a plurality of types of fibers made of different materials, or the cover layer 31 may be configured by laminating a plurality of non-woven fabrics.

The method for producing the non-woven fabric is not particularly limited, and any of a spunlace non-woven fabric, a thermal bond non-woven fabric, a spunbond non-woven fabric, a needle punched non-woven fabric, and the like can be applied to the present invention.

The basis weight of the non-woven fabric is preferably about 10 to 200 g/m$^2$, more preferably about 30 to 150 g/m$^2$, and still more preferably about 50 to 100 g/m$^2$.

The thickness of the non-woven fabric is preferably about 0.01 to 3 mm, more preferably about 0.1 to 2 mm, still more preferably about 0.3 to 1 mm, and particularly preferably about 0.5 to 0.86 mm.

The elongation rate of the non-woven fabric in both the flow direction and the width direction is 50% to 300%, and still more preferably 68% to 269%. The average of the elongation rates in the flow direction and the width direction of the non-woven fabric is 50% to 300%, still more preferably 100% to 250%, and most preferably 102% to 207%.

The elongation recovery rate of the non-woven fabric in both the flow direction and the width direction is preferably 50% to 85% and more preferably 50% to 70%.

The "flow direction" is a direction in which the non-woven fabric is unwound or wound up in the case where the non-woven fabric is continuously produced by a machine, and the "width direction" is a direction orthogonal to the flow direction. In the case of a rectangular non-woven fabric that is in circulation, a certain side is usually the flow direction, and a direction orthogonal to that side is the width direction. In the present invention, since the suitable numerical range of the elongation rate is the same in the flow direction and the width direction, for example, in the case where the cover layer has a non-rectangular shape such as a circle, the elongation rate in both a certain direction and a direction orthogonal thereto may be within a suitable numerical range.

The cover adhesive layer 32 according to the present embodiment is made of a rubber-based adhesive in which the main adhesive base is a rubber-based adhesive base. Here, the phrase "in which the main adhesive base is a rubber-based adhesive base" indicates that the proportion of the rubber-based adhesive base is the largest based on the total mass of the adhesive base of the cover adhesive layer 32. In the present invention, the cover adhesive layer 32 preferably does not contain an adhesive base other than the rubber-based adhesive base, but may contain such an adhesive base. Examples of the adhesive base other than the rubber-based adhesive base include an acrylic adhesive base and a silicone-based adhesive base, which are exemplary examples as the adhesive bases for the drug layer 11.

Examples of the material for the rubber-based adhesive base in the cover adhesive layer 32 include polyisobutylene, polybutene, polyisoprene, polybutadiene, a styrene-isoprene copolymer, a styrene-isoprene-styrene block copolymer, a styrene-butadiene copolymer, a styrene-butadiene-styrene block copolymer, an alkyl vinyl ether (co)polymer, and a natural rubber. These compounds can be used alone or in combination of two or more thereof.

From the viewpoint that it is difficult for drugs and additives contained in the drug layer 11 to migrate to the cover adhesive layer 32, it is preferable that polyisobutylene, polybutene, polyisoprene, and polybutadiene be the main components among the above-described compounds, and it is more preferable that polyisobutylene be the main component.

Among rubber-based adhesive bases, specific examples of the styrene-isoprene-styrene block copolymer include Quintac (registered trademark) 3570C (trade name, manufactured by Zeon Corporation), SIS5002, SIS5229, and SIS5505 (trade names, manufactured by JSR Corporation), and SIBSTAR (registered trademark) T102 (trade name, manufactured by Kaneka Corporation). The polyisobutylene also includes a so-called butyl rubber (isobutylene-isoprene rubber). Specific examples of a polyisobutylene include Oppanol (registered trademark) N50, N80, N100, N150, B11, B12, B50, B80, B100, B120, B150, and B220 (trade names, manufactured by BASF SE), JSR (registered trademark) Butyl 065, 268, and 365 (trade names, manufactured by JSR Corporation), X_Butyl (registered trademark) RB100, 101-3, 301, and 402 (trade names, manufactured by Arlanxeo Company), Exxon (registered trademark) Butyl 065, 065S, 068, 068S, 268, 268S, 365, and 365S (trade names, manufactured by Exxon Mobil Corporation), and Butyl 065, 268, and 365 (trade names, manufactured by Nippon Butyl Co., Ltd.).

Since the cover adhesive layer 32 consists of a rubber-based adhesive in which the main adhesive base is a rubber-based adhesive base, the content of the rubber-based adhesive base is preferably 50% by mass or more, more preferably 70% by mass or more, still more preferably 90% by mass or more, particularly preferably 95% by mass or more, and most preferably 100% by mass, based on the total mass of the adhesive base of the cover adhesive layer 32.

Furthermore, the content of at least one selected from the group consisting of polyisobutylene, polybutene, polyisoprene, and polybutadiene among the rubber-based adhesive bases is preferably 50% by mass or more, more preferably 70% by mass or more, still more preferably 90% by mass or more, particularly preferably 95% by mass or more, and most preferably 100% by mass, based on the total mass of the rubber-based adhesive base of the cover adhesive layer 32.

The cover adhesive layer 32 may contain an additive other than the rubber-based adhesive base. Examples of the additive include a tackifier resin and a plasticizer, and one additive may be used alone or two or more additives may be used in combination.

The mass of the cover adhesive layer 32 is preferably about 5 to 300 $g/m^2$, more preferably about 30 to 200 $g/m^2$, and still more preferably 50 to 150 $g/m^2$. In the case where the mass of the cover adhesive layer is less than the above-described lower limit, the adhesiveness tends to decrease, and in the case where the mass of the cover adhesive layer is more than the above-described upper limit, it tends to be difficult to maintain a sufficient thickness or shape.

The cover adhesive layer 32 protruding around the drug layer 11 and the adhesive patch part 10 is covered and protected by a release liner 2 until use.

In the case where the adhesive patch 1 with a cover material is used, the release liner 2 is peeled off, and the exposed drug layer 11 and cover adhesive layer 32 are brought into contact with and attached to the user's skin.

The material for the release liner 2 is not particularly limited, and various known liners can be used. Examples of the material for the release liner 2 include paper; polyesters such as PET and polyethylene naphthalate; polyolefins such as polyethylene and polypropylene; polyvinyl chloride, polyvinylidene chloride, nylon, and aluminum. A film made of PET is particularly preferable. The release liner 2 may be made of a plurality of materials, such as a laminated film of high-quality paper and polyolefin. The surface of the release liner in contact with the drug layer 11 and the cover adhesive layer 32 is preferably subjected to a release treatment with silicone, fluorinated polyolefin, or the like.

Persons having ordinary skill in the art recognize several problems in an adhesive patch with a cover material in the related art.

One is the migration of a drug contained in a drug layer of the adhesive patch to an adhesive layer of the cover material. In the case where an excessive amount of the drug migrates to the adhesive layer, there is a possibility that the drug may not reach a target blood concentration in the user's body, or may not be able to maintain the blood concentration for a target period of time.

The other is the occurrence of stickiness due to the protrusion of the adhesive layer of the cover material. This is a phenomenon in which the adhesive layer of the cover material adheres to the outside of the region where the adhesive patch with a cover material is first applied, and is also referred to as "tongue-sticking out". The tongue-sticking out that remains after the adhesive patch with a cover material is peeled off after the end of the medication period is referred to as "adhesive residue". The occurrence of the adhesive residue causes discomfort to the user.

The present inventors conducted various studies to resolve these problems. As a result, it has been found that the above problems can be solved by adopting the following configuration.

· Making the cover layer of a non-woven fabric, and setting the elongation rate in the flow direction and the width direction to 50% to 300%.

· Making the cover adhesive layer of a rubber-based adhesive in which the main adhesive base is a rubber-based adhesive base, and the loss tangent at 32°C and 0.1 Hz is set to 0.7 to 0.95.

First, regarding the migration of a drug to the adhesive layer of the cover material, it was found that the migration of the drug to the adhesive layer of the cover material was much less with the rubber-based adhesive in which the main adhesive base was a rubber-based adhesive base, compared with the commonly used acrylic adhesive in the related art.

Next, regarding the adhesive residue, based on the hypothesis that the difference in followability between the cover layer and the cover adhesive layer plays an important role in responding to the displacement of the application surface due to the body movement or the like of the user of the adhesive patch with a cover material, the present inventors have conducted various studies shown below. As a result, the present inventors arrived at the optimum configuration of the cover layer.

Furthermore, regarding the loss tangent of the cover adhesive layer, it was found that, in the case where the loss tangent of the cover adhesive layer was too small, the adhesiveness decreased and the adhesive patch with a cover material tended to peel off easily during application; and in the case where the loss tangent of the cover adhesive layer was too large, the adhesive tended to remain after peeling off of the adhesive patch with a cover material due to the tongue-sticking out during application. The range of the loss tangent was preferably 0.75 to 0.90, and most preferably 0.80 to 0.90.

Hereinafter, the results of studies that served as a basis for various configurations of the adhesive patch with a cover material according to the present embodiment will be described.

Only the samples falling within the scope of the claims are according to the invention, while the others are provided for illustrative purposes.

(Experiment 1)

In Experiment 1, the composition of the cover adhesive layer and the migration of components in the drug layer were investigated.

The components in the amounts shown below were thoroughly mixed in an appropriate amount of a solvent to prepare a composition to form an experimental drug layer. This composition was uniformly spread on a release liner (a film made of polyethylene terephthalate that had been subjected to a release treatment; the same shall apply hereinafter) and the solvent was removed by drying to form an experimental drug layer 1 having a mass of the paste per unit area of 80 g/m$^2$. A backing consisting of a PET film was attached to the surface of the obtained experimental drug layer opposite to the release liner, and the resulting structure was cut to 2.5 cm$^2$ to prepare an experimental adhesive patch part.

(Experimental drug layer 1)

| | |
|---|---|
| Rubber-based adhesive base (containing styrene-isoprene-styrene block copolymer and polyisobutylene in a mass ratio of 1:1) | 23.38 parts by mass |
| Silicone-based adhesive base (BIO-PSA (registered trademark) AC7-4201 Silicone Adhesive, manufactured by Dupont Toray Specialty Materials K.K.) | 7.19 parts by mass |
| Terpene-based resin | 26.97 parts by mass |
| Liquid paraffin | 14.38 parts by mass |
| Butorphanol tartrate | 6 parts by mass |
| Oleyl alcohol | 10 parts by mass |
| Other components (adsorbent and desalting agent) | 12.08 parts by mass |

Next, each component of the cover adhesive layer shown in Table 1 was thoroughly mixed in an appropriate amount of a solvent to prepare a composition to form a cover adhesive layer. This composition was uniformly spread on a release liner, and the solvent was removed by drying to form a cover adhesive layer having a mass per unit area of 75 g/m$^2$. A cover layer consisting of a PET film was attached to the surface of the obtained cover adhesive layer opposite to the release liner, and then the resulting structure was cut to 5 cm$^2$ to prepare an experimental cover part according to each sample.

Details of the materials shown in Table 1 are described below.

Rubber-based adhesive 1:

Adhesive containing styrene-isoprene-styrene block copolymer/polyisobutylene/alicyclic saturated hydrocarbon resin/liquid paraffin in a mass ratio of 16.3:7:51.1:18.6. Since the alicyclic saturated hydrocarbon resin and the liquid paraffin were additives, the rubber-based adhesive 1 contained only a rubber-based adhesive base as the adhesive base.

Acrylic adhesive 1: MAS811B (manufactured by CosMED Pharmaceutical Co., Ltd.)

Acrylic adhesive 2: DURO-TAK (registered trademark) 87-2051 (manufactured by Henkel AG & Co. KGaA)

Acrylic adhesive 3: DURO-TAK (registered trademark) 87-2194 (manufactured by Henkel AG & Co. KGaA)

Acrylic adhesive 4: DURO-TAK (registered trademark) 87-2516 (manufactured by Henkel AG & Co. KGaA)

An adhesive patch with a cover material according to each sample was obtained by peeling off the release liner of the experimental cover part and attaching the experimental cover part to the backing side of the experimental adhesive patch part to completely cover the experimental adhesive patch part in plan view.

[Table 1]

| | SAMPLE1 | SAMPLE2 | SAMPLE3 | SAMPLE4 | SAMPLE5 | SAMPLE6 |
|---|---|---|---|---|---|---|
| COVER ADHESIVE LAYER | POLYSOBUTYLENE | RUBBER-BASED ADHESIVE1 | ACRYLIC ADHESIVE1 | ACRYLIC ADHESIVE2 | ACRYLIC ADHESIVE3 | ACRYLIC ADHESIVE4 |
| PERCENT MIGRATION OF BUTORPHANOL (%) | 0.1 | 0.4 | 1.9 | 4.2 | 0.9 | 1.0 |
| PERCENT MIGRATION OF OLEYL ALCOHOL (%) | 3.2 | 12.0 | 17.0 | 29.0 | 19.8 | 19.2 |

In each sample, the migration of components to the cover adhesive layer was evaluated by the following procedure.

The adhesive patch with a cover material according to each sample was enclosed in a packaging bag and stored at 60°C for two weeks. Thereafter, the adhesive patch with a cover material was removed from the packaging bag, the release liner was peeled off, and the experimental adhesive patch part was separated from the experimental cover part. The amounts of butorphanol and oleyl alcohol contained in the cover adhesive layer of the experimental cover part were measured to calculate the migration rate from the experimental drug layer.

As shown in Table 1, in Samples 1 and 2 in which the cover adhesive layer was formed with a rubber-based adhesive in which the main adhesive base was a rubber-based adhesive base, the migration of butorphanol to the cover adhesive layer was suppressed as compared with Samples 3 to 6, which used an acrylic adhesive in which the main adhesive base was an acrylic adhesive base. In particular, in Sample 1, in which the cover adhesive layer consisted of polyisobutylene, the migration rate of oleyl alcohol was also suppressed to a low level.

From the above, the superiority of using the rubber-based adhesive in which the main adhesive base was a rubber-based adhesive base in the cover adhesive layer was confirmed.

(Experiment 2)

In Experiment 2, the adhesive residue that caused stickiness due to the cover adhesive layer was examined.

The components in the amounts shown below were thoroughly mixed in an appropriate amount of a solvent to prepare a composition to form an experimental drug layer. This composition was uniformly spread on a release liner, and the solvent was removed by drying to form an experimental drug layer 2 having a mass of the paste per unit area of 60 g/m$^2$. A backing consisting of a PET film was attached to the surface of the obtained experimental drug layer opposite to the release liner, and the resulting structure was cut to 30 cm$^2$ to prepare an experimental adhesive patch part.

(Experimental drug layer 2)

| | |
|---|---|
| Rubber-based adhesive base (containing styrene-isoprene-styrene block copolymer and polyisobutylene in a mass ratio of 1:1) | 24.88 parts by mass |
| Silicone-based adhesive base (BIO-PSA (registered trademark) AC7-4201 Silicone Adhesive) | 7.65 parts by mass |
| Terpene-based resin | 28.70 parts by mass |
| Liquid paraffin | 15.31 parts by mass |
| Tartaric acid | 1.89 parts by mass |
| Oleyl alcohol | 10 parts by mass |
| Other components (adsorbent and pH-adjusting agent) | 11.57 parts by mass |

Next, materials according to the composition of the cover adhesive layer described in Tables 2 to 5 were thoroughly mixed in an appropriate amount of a solvent to prepare a composition to form a cover adhesive layer. This composition was uniformly spread on a release liner, and the solvent was removed by drying to form a cover adhesive layer having a mass per unit area of 75 g/m$^2$. A cover layer having a configuration described in Tables 2 to 5 was attached to the surface of the obtained cover adhesive layer opposite to the release liner, and then the resulting structure was cut to 47.6 cm$^2$ to prepare an experimental cover part according to each sample. Regarding the non-woven fabrics and knitted fabrics described in each table, the same descriptions mean that the non-woven fabric or knitted fabric was the same non-woven fabric or knitted fabric.

An adhesive patch with a cover material according to each sample was obtained by peeling off the release liner of the experimental cover part and attaching the experimental cover part to the backing side of the experimental adhesive patch part to completely cover the experimental adhesive patch part in plan view.

The following evaluations were carried out for each sample.

(Calculation of loss tangent)

In each sample, the cover adhesive layer was peeled off from the cover layer, and dynamic viscoelasticity measurement was carried out under the following measurement conditions using a rheometer "HAAKE MARS" (manufactured by Thermo Fisher Scientific Inc.). The loss tangent of the cover adhesive layer according to each sample was calculated by the following expression using the measured values of the loss elastic modulus and the storage elastic modulus.

$$\text{Loss tangent (tan}\delta) = \text{loss elastic modulus (G")/storage elastic modulus (G')}$$

[Measurement conditions]

· Sample part: flat plate with a diameter of 8 mm
· Gap interval: 1 mm
· Sample amount: 0.1 to 0.5 g
· Temperature: 32°C
· Frequency: 0.1 Hz
· Strain: 1%

(Evaluation of adhesive properties)

After the adhesive patch with a cover material according to each sample was applied to the upper arm part of each of 20 subjects, who were then allowed to behave in the same manner as usual for 24 hours, the adhesive properties of the cover part were scored based on the following standards and an average score for each sample was obtained. The degree of peeling in the following standards is a ratio assuming that the area of the portion of the cover adhesive layer which is not attached to the adhesive patch part (that is, the portion in contact with the skin of the subject) is set to 100.

No peeling: 100
Approximately 1/10 (less than 1/8) peeling: 80
Approximately 1/5 (1/8 or more and less than 1/4) peeling: 60
Approximately 1/3 (1/4 or more and less than 1/2) peeling: 40
1/2 or more peeling: 20
Dropout: 0

Furthermore, the average scores were ranked as follows, and only A grade was regarded as acceptable.

A: Average score of 80 or more
B: Average score of 60 or more and less than 80
C: Average score of less than 60

(Evaluation of adhesive residue)

After the evaluation of the adhesive properties, the adhesive patch with a cover material was peeled off. The adhesive residue after peeling was scored based on the following standards, and an average score for each sample was obtained.

No adhesive residue was observed: 100
Adhesive residue was observed in approximately 1/10 (less than 1/8) of the periphery of the cover part: 80
Adhesive residue was observed in approximately 1/4 (1/8 or more and less than 3/8) of the periphery of the cover part: 60
Adhesive residue was observed in approximately 1/2 (3/8 or more and less than 5/8) of the periphery of the cover part: 40
Adhesive residue was observed in approximately 3/4 (5/8 or more) of the periphery of the cover part: 20
Adhesive residue was observed on the entire periphery of the cover part: 0

Furthermore, the average scores were ranked as follows, and only A grade was regarded as acceptable.

A: Average score of 80 or more
B: Average score of 60 or more and less than 80
C: Average score of less than 60

[Table 2]

|  |  | SAMPLE 11 | SAMPLE 12 | SAMPLE 13 |
|---|---|---|---|---|
| COVER ADHESIVE LAYER | COMPOSITION | POLYISOBUTYLENE | POLYISOBUTYLENE | POLYISOBUTYLENE |
|  | LOSS TANGENT | 0.86 | 0.86 | 0.86 |
| COVER LAYER | FORM (MATERIAL) | NON-WOVEN FABRIC (PET) | KNITTED FABRIC (PET) | FILM (PET) |
| EVALUATION | ADHESIVE RESIDUE (AVERAGE SCORE/RANK) | 95/A | 69/B | 56/C |
|  | ADHESIVE PROPERTIES (AVERAGE SCORE/RANK) | 93/A | 97/A | 86/A |

[Table 3]

|  |  | SAMPLE 14 | SAMPLE 15 | SAMPLE 11 | SAMPLE 16 | SAMPLE 17 | SAMPLE 18 |
|---|---|---|---|---|---|---|---|
| COVER ADHESIVE LAYER | COMPOSITION | POLYISOBUTYLENE | POLYISOBUTYLENE | POLYISOBUTYLENE | POLYISOBUTYLENE | POLYISOBUTYLENE | POLYISOBUTYLENE |
|  | LOSS TANGENT | 1.00 | 0.95 | 0.86 | 0.75 | 0.60 | 0.40 |
| COVER LAYER | FORM (MATERIAL) | NON-WOVEN FABRIC (PET) | NON-WOVEN FABRIC (PET) | NON-WOVEN FABRIC (PET) | NON-WOVEN FABRIC (PET) | NON-WOVEN FABRIC (PET) | NON-WOVEN FABRIC (PET) |
| EVALUATION | ADHESIVE RESIDUE (AVERAGE SCORE/RANK) | 66/B | 84/A | 95/A | 86/A | 89/A | 89/A |
|  | ADHESIVE PROPERTIES (AVERAGE SCORE/RANK) | 90/A | 90/A | 93/A | 94/A | 77/B | 56/C |

[Table 4]

|  |  | SAMPLE 19 | SAMPLE 12 | SAMPLE 20 |
|---|---|---|---|---|
| COVER ADHESIVE LAYER | COMPOSITION | POLYISOBUTYLENE | POLYISOBUTYLENE | POLYISOBUTYLENE |
|  | LOSS TANGENT | 0.95 | 0.86 | 0.75 |
| COVER LAYER | FORM (MATERIAL) | KNITTED FABRIC (PET) | KNITTED FABRIC (PET) | KNITTED FABRIC (PET) |
| EVALUATION | ADHESIVE RESIDUE (AVERAGE SCORE/RANK) | 63/B | 69/B | 73/B |
|  | ADHESIVE PROPERTIES (AVERAGE SCORE/RANK) | 98/A | 97/A | 94/A |

[Table 5]

| | | SAMPLE 21 | SAMPLE 22 |
|---|---|---|---|
| COVER ADHESIVE LAYER | COMPOSITION | POLYISOBUTYLENE/ STYRENE-ISOPRENE-STYRENE BLOCK COPOLYMER | POLYISOBUTYLENE/ STYRENE-ISOPRENE-STYRENE BLOCK COPOLYMER |
| | LOSS TANGENT | 0.87 | 0.87 |
| COVER LAYER | FORM (MATERIAL) | NON-WOVEN FABRIC (PET) | KNITTED FABRIC (PET) |
| EVALUATION | ADHESIVE RESIDUE (AVERAGE SCORE/RANK) | 84/A | 67/B |
| | ADHESIVE PROPERTIES (AVERAGE SCORE/RANK) | 89/A | 93/A |

The results of the samples shown in Table 2 indicated that the non-woven fabric is suitable as a cover layer.

The loss tangent is an index that shows the balance between elasticity and viscosity of a substance. Each sample shown in Table 3 was prepared using various polyisobutylenes having different loss tangents. The results of the samples shown in Table 3 indicated that the loss tangent of the cover adhesive layer was preferably about 0.7 to 0.95.

From the results of the samples shown in Table 4, it was confirmed that, even in the case where the loss tangent of the cover adhesive layer was within the above-described suitable range, an effect of suppressing adhesive residue was not sufficient in the case where the cover layer was a knitted fabric (a cloth in which fibers are knitted); that is, the cover adhesive layer having a loss tangent within the above-described suitable range exhibited a favorable effect of suppressing adhesive residue only in the case of being combined with a cover layer consisting of a non-woven fabric.

From the results of the samples shown in Table 5, it was confirmed that, even in the case of the cover adhesive layer in which a styrene-isoprene-styrene block copolymer was mixed as the rubber-based adhesive base other than polyisobutylene, a favorable effect was exhibited of suppressing adhesive residue in the case of having a loss tangent within the above-described suitable range and being combined with a cover layer consisting of a non-woven fabric. The ratio of polyisobutylene to styrene-isoprene-styrene block copolymer in the cover adhesive layers of Samples 21 and 22 was 9:1, and polyisobutylene was the main component.

(Experiment 3)

In Experiment 3, since a non-woven fabric was suitable as the cover layer, suitable physical properties of the non-woven fabric were examined.

Samples 31 to 35 were prepared in which only the non-woven fabric serving as the cover layer was changed from Sample 11 used in Experiment 2.

The following measurements were carried out on the non-woven fabrics used as the cover layers of Samples 11 and 31 to 35.

(Measurement of elongation rate)

The measurement of elongation rate was carried out in accordance with 8.14.1A described in JIS L 1096:2010.

The non-woven fabric used in each sample was sampled along the flow direction and the width direction to have a size of 25 mm in width and 110 mm in length, and used as a test piece. The test piece was gripped by a tensile tester so that a grip interval in the length direction was 60 mm and pulled at a tensile speed of 500 mm/min. Then, the elongation rate (%) was measured from the length at cutting ($L_1$) and the original length ($L_0$) using the following expression. Each non-woven fabric was measured three times in the flow direction and in the width direction, and the average of the measured values was taken as the value of the elongation rate.

$$\text{Elongation rate (\%)} = (L_1 - L_0) \times 100/L_0$$

(Measurement of elongation recovery rate)

The measurement of elongation recovery rate was carried out in accordance with 8.15.1A described in JIS L 1096:2010.

For the non-woven fabric used in each sample, a test piece similar to that used for the measurement of elongation rate was prepared. The test piece was gripped by a tensile tester so that a grip interval in the length direction was 60 mm and stretched at a tensile speed of 500 mm/min to a length ($L_1$) that is 150% of the original length ($L_0$)), and the load was removed at the same speed. The elongation recovery rate (%) was calculated from the following expression, setting the length at the time when the stress became 0 as $L_2$. Each non-woven fabric was measured three times, and the average of the measured values was taken as the value of the elongation recovery rate.

$$\text{Elongation recovery rate } (\%) = (L_1 - L_2) \times 100/(L_1 - L_0)$$

For Samples 31 to 35, the evaluation of adhesive properties and the evaluation of adhesive residue, which were carried out in Experiment 2, were also carried out.

All results are shown in Table 6. The material for the non-woven fabric of Sample 35 was polyurethane.

The non-woven fabric of Sample 31 was torn without being stretched to a length that was 150% of the original length, so the elongation recovery rate could not be measured.

[Table 6]

| | | SAMPLE 31 | SAMPLE 32 | SAMPLE 33 | SAMPLE 11 | SAMPLE 34 | SAMPLE 35 |
|---|---|---|---|---|---|---|---|
| COVER ADHESIVE LAYER | COMPOSITION | POLYISOBUTYLENE | POLYISOBUTYLENE | POLYISOBUTYLENE | POLYISOBUTYLENE | POLYISOBUTYLENE | POLYISOBUTYLENE |
| | LOSS TANGENT | 0.86 | 0.86 | 0.86 | 0.86 | 0.86 | 0.86 |
| COVER LAYER | FORM (MATERIAL) | NON-WOVEN FABRIC(PET) | NON-WOVEN FABRIC(PET) | NON-WOVEN FABRIC(PET) | NON-WOVEN FABRIC(PET) | NON-WOVEN FABRIC(PET) | NON-WOVEN FABRIC(PU) |
| | BASIS WEIGHT ($g/m^2$) | 15 | 100 | 50 | 84 | 100 | 75 |
| | THICKNESS (cm) | 0.044 | 0.5 | 0.62 | 0.61 | 0.86 | 0.33 |
| | ELONGATION RATE(%) FLOW DIRECTION/ WIDTH DIRECTION | 21/7 | 68/136 | 134/171 | 145/269 | 185/227 | 475/466 |
| | ELONGATION RECOVERY RATE(%) FLOW DIRECTION/ WIDTH DIRECTION | -/- | 23/43 | 63/59 | 70/68 | 75/82 | 89/90 |
| EVALUATION | ADHESIVE RESIDUE (AVERAGE SCORE/RANK) | 74/B | 92/A | 89/A | 95/A | 83/A | 78/B |
| | ADHESIVE PROPERTIES (AVERAGE SCORE/RANK) | 85/A | 93/A | 93/A | 93/A | 87/A | 90/A |

From the results in Table 6, the following findings were obtained.

· In the case where the elongation rate in the flow direction and the width direction of the non-woven fabric used as the cover layer was within a range of 50% to 300%, adhesive residue could be suitably suppressed in combination with the cover adhesive layer having the above-described loss tangent.

· In the case where the elongation recovery rate in the flow direction and the width direction of the non-woven fabric used as the cover layer was within a range of 20% to 85%, adhesive residue could be suitably suppressed in combination with the cover adhesive layer having the above-described loss tangent.

The adhesive patch 1 with a cover material of the present embodiment, in which the specific configuration of each part is set based on the findings obtained from each of the above-described experiments, suitably maintains a state in which the drug layer 11 is in contact with the skin due to the favorable adhesive properties of the cover part 30, and suppresses adhesive residue on the cover part 30, thereby suppressing unpleasant stickiness during use. Further, since the cover adhesive layer is made of a rubber-based adhesive in which the main adhesive base is a rubber-based adhesive base, migration of the drug contained in the drug layer 11 to the cover adhesive layer is also suppressed, which also has an advantage of facilitating formulation design.

Industrial Applicability

The present invention can be suitably applied to an adhesive patch with a cover material.

Description of Reference Signs

    1: adhesive patch with cover material
    10: adhesive patch part
    11: drug layer
    12: backing
    30: cover part
    31: cover layer
    32: cover adhesive layer

**Claims**

1.  An adhesive patch with a cover material, comprising:

    an adhesive patch part (10) including a backing (12) and a drug layer (11) formed on the backing (12); and
    a cover part (30) including a cover layer (31) and a cover adhesive layer (32) formed on the cover layer (31), wherein
    the cover adhesive layer (32) is attached to the backing (12) to cover the adhesive patch part (10),
    the cover layer (31) is formed of a non-woven fabric, and has an elongation rate of 50% to 300% in a flow direction and a width direction, and
    the cover adhesive layer (32) is made of a rubber-based adhesive in which a main adhesive base is a rubber-based adhesive base, and has a loss tangent (tan$\delta$) of 0.7 to 0.95 at 32°C and 0.1 Hz.

2.  The adhesive patch with a cover material according to claim 1, wherein the rubber-based adhesive base contains at least one selected from the group consisting of polyisobutylene, polybutene, polyisoprene, and polybutadiene as a main component.

3.  The adhesive patch with a cover material according to claim 1 or 2, wherein the rubber-based adhesive base consists of one or more selected from the group consisting of polyisobutylene, polybutene, polyisoprene, and polybutadiene.

4.  The adhesive patch with a cover material according to one of claims 1 to 3, wherein the non-woven fabric has an elongation recovery rate of 85% or less in the flow direction and the width direction.

**Patentansprüche**

1.  Klebepflaster mit Abdeckmaterial, umfassend:

    ein Klebepflasterteil (10) aufweisend einen Träger (12) und eine Wirkstoffschicht (11), die auf dem Träger (12) ausgebildet ist; und
    ein Abdeckteil (30) aufweisend eine Abdeckschicht (31) und eine Abdeckklebeschicht (32), die auf der Abdeckschicht (31) ausgebildet ist, wobei
    die Abdeckklebeschicht (32) an dem Träger (12) angebracht ist, um das Klebepflasterteil (10) abzudecken,
    die Abdeckschicht (31) aus einem Vliesstoff ausgebildet ist und eine Dehnungsrate von 50% bis 300% in einer Fließrichtung und einer Breitenrichtung aufweist, und
    die Abdeckklebeschicht (32) aus einem Klebstoff auf Kautschukbasis gemacht ist, bei dem eine Hauptklebstoffbasis eine Klebstoffbasis auf Kautschukbasis ist, und einen Verlustfaktor (tan$\delta$) von 0,7 bis 0,95 bei 32°C und 0,1 Hz aufweist.

2. Klebepflaster mit Abdeckmaterial gemäß Anspruch 1, wobei die Klebstoffbasis auf Kautschukbasis mindestens eines ausgewählt aus der Gruppe bestehend aus Polyisobutylen, Polybuten, Polyisopren und Polybutadien als eine Hauptkomponente enthält.

3. Klebepflaster mit Abdeckmaterial gemäß Anspruch 1 oder 2, wobei die Klebstoffbasis auf Kautschukbasis aus einem oder mehreren ausgewählt aus der Gruppe bestehend aus Polyisobutylen, Polybuten, Polyisopren und Polybutadien besteht.

4. Klebepflaster mit Abdeckmaterial gemäß einem der Ansprüche 1 bis 3, wobei der Vliesstoff eine Dehnungserholungsrate von 85% oder weniger in der Fließrichtung und der Breitenrichtung aufweist.

**Revendications**

1. Patch adhésif avec un matériau de couverture, comprenant :

une partie de patch adhésif (10) comprenant un support (12) et une couche de médicament (11) formée sur le support (12) ; et
une partie de couverture (30) comprenant une couche de couverture (31) et une couche adhésive de couverture (32) formée sur la couche de couverture (31), dans lequel
la couche adhésive de couverture (32) est fixée au support (12) pour couvrir la partie de patch adhésif (10),
la couche de couverture (31) est formée d'un tissu non tissé, et a un taux d'allongement de 50 % à 300 % dans une direction d'écoulement et une direction de largeur, et
la couche adhésive de couverture (32) est constituée d'un adhésif à base de caoutchouc dans lequel une base adhésive principale est une base adhésive à base de caoutchouc, et a un tangente de perte (tan$\delta$) de 0,7 à 0,95 à 32 °C et 0,1 Hz.

2. Patch adhésif avec un matériau de couverture selon la revendication 1, dans lequel la base adhésive à base de caoutchouc contient au moins un choisi dans le groupe constitué de polyisobutylène, de polybutène, de polyisoprène, et de polybutadiène en tant que composant principal.

3. Patch adhésif avec un matériau de couverture selon la revendication 1 ou 2, dans lequel la base adhésive à base de caoutchouc est constituée d'un ou plusieurs choisis dans le groupe constitué de polyisobutylène, de polybutène, de polyisoprène, et de polybutadiène.

4. Patch adhésif avec un matériau de couverture selon l'une des revendications 1 à 3, dans lequel le tissu non tissé a un taux de récupération d'allongement de 85 % ou moins dans la direction d'écoulement et la direction de largeur.

# FIG. 1

$\underline{1}$

10

30

I

I

# FIG. 2

$\underline{1}$

30

10

32

2

31

11   12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021180374 A
- JP H11343232 A

- EP 3263097 A